(12)  **EUROPEAN PATENT SPECIFICATION**

(45) Date of publication and mention
of the grant of the patent:
**16.04.2003  Bulletin 2003/16**

(21) Application number: **98930598.2**

(22) Date of filing: **30.06.1998**

(51) Int Cl.[7]: **A61K 31/715**, A61P 11/10

(86) International application number:
**PCT/CA98/00628**

(87) International publication number:
**WO 99/001141 (14.01.1999 Gazette 1999/02)**

(54) **USE OF DEXTRAN AND OTHER NON-SULFATED POLYSACCHARIDES TO IMPROVE MUCUS CLEARANCE**

VERWENDUNG VON DEXTRAN UND WEITEREN NICHT-SULFATIERTEN POLYSACCHARIDEN ZUR FÖRDERUNG DER MUKUSKLÄRUNG

UTILISATION DU DEXTRAN ET D'AUTRES POLYSACCHARIDES NON SULFATES POUR AMELIORER L'EPURATION DES MUCOSITES

(84) Designated Contracting States:
**AT BE CH CY DE DK ES FI FR GB GR IE IT LI LU MC NL PT SE**
Designated Extension States:
**AL LT LV MK RO SI**

(30) Priority: **30.06.1997  CA 2209342**
**31.03.1998  CA 2233805**

(43) Date of publication of application:
**29.03.2000  Bulletin 2000/13**

(73) Proprietors:
• **THE UNIVERSITY OF BRITISH COLUMBIA**
**Vancouver, British Columbia V6T 1Z3 (CA)**
• **THE GOVERNORS OF THE UNIVERSITY OF ALBERTA**
**Edmonton, Alberta T6G 2E1 (CA)**

(72) Inventors:
• **KING, Malcolm**
**Edmonton, Alberta T6J 3J2 (CA)**
• **SPEERT, David, P.**
**Vancouver, British Columbia V6R 1L1 (CA)**

(74) Representative: **Senior, Janet et al**
**Abel & Imray**
**20 Red Lion Street**
**London WC1R 4PQ (GB)**

(56) References cited:
**WO-A-91/15216**          **WO-A-95/17898**

• **W. FENG ET AL.: "Improved clearability of cystic fibrosis sputum with dextran treatment." CLIN. INVEST. MED, vol. 20, no. 4 suppl., 1997, page S99 XP002084085**
• **W. FENG ET AL.: "Improved clearability of cystic fibrosis sputum with dextran treatment in vitro." AM. J. RESPIR. CRIT. CARE MED., vol. 157, no. 3, 1998, pages 710-714, XP002084086**

EP 0 988 041 B1

**Description**

FIELD OF THE INVENTION

This invention relates to means for improving mucus clearance, and more particularly, the invention relates to the use of a non-sulfated polysaccharide such as dextran in the manufacture of a medicament to improve mucus clearance.

BACKGROUND OF THE INVENTION

Mucus is a critical component of the primary defence mechanism of the respiratory tract, trapping inhaled particulate and microbial material for removal via the mucociliary system. When this mechanism fails to clear sufficiently, mucus accumulates, and must be coughed up as sputum; otherwise it is retained in the respiratory tract, encouraging colonization by microorganisms, which may lead to chronic lung inflammation and obstruction. In cystic fibrosis (CF), airway mucus obstruction has long been considered the most insidious agent of morbidity and mortality. Therapies designed to thin the airway mucus and improve its clearability continue to be a major focus of attention.

Airway mucus is a complex, viscoelastic gel whose physical properties are important for airway defence. Mucus is a variable mixture of water, mucous glycoproteins, low molecular weight ions, proteins, and lipids. The three-dimensional structure that forms the mucous gel is dependent upon a number of forms of bonding. The main elements include the following: 1) disulfide bonds - these covalent links are mainly intramolecular, and join glycoprotein subunits into extended macromolecular chains known as mucins. 2) Because of their extended size, these mucin polymers readily form entanglements with neighbouring macromolecules; these act as time-dependent crosslinks, which are susceptible to mechanical degradation. 3) The sugar units that make up the oligosaccharide side-chains (about 80% of the mucin weight), form hydrogen bonds with complimentary units on neighbouring mucins. Although each bond is weak and readily dissociates, the numbers of bond sites make this type of bonding potentially very important. 4) Mucins are also ionized, containing both positively charged amino acid residues as well as negatively charged sugar units, principally sialic acid and sulfated residues. These increase in airway disease in general, and in CF the proportion of sulfated residues is further elevated because of alterations in glycosyl transferase activities within the Golgi apparatus. The ionic interactions between fixed negative charges result in a stiffer, more extended macromolecular conformation, effectively increasing the polymer size and adding to the numbers of entanglements. 5) Added to this in airway diseases characterized by infection and inflammation, especially CF, are the extra networks of high molecular weight DNA and actin filaments released by dying leukocytes, and exopolysaccharides secreted by bacteria.

One of the primary aspects of the current treatment of CF lung disease is aimed at changing the physical properties of pulmonary secretions to improve their clearance from the airways. The most successful therapy in CF, and the only mucoactive agent with proven efficacy, is rhDNase. Treatment with rhDNase is based on the fact that the major factor involved in the elevated viscoelasticity of CF sputum is attributed to the presence of naked DNA released into the airway surface fluid (ASF) from bacteria, neutrophils, and other cellular debris. Enzymatic digestion of these DNA macromolecules effectively decreases mucus viscoelasticity and spinnability and enhances the clearability of airway secretions. Other direct-acting mucolytic treatments, such as N-acetylcysteine derivatives, gelsolin, and hypertonic saline, are effective in vitro in CF, but may not necessarily show clinical efficacy. Indirect mucolysis, such as with inhaled amiloride, which blocks the uptake of salt and water across the airway epithelium, is a strategy aimed at enhancing the degree of hydration and diluting the macromolecular component of the ASF. Combined mucokinetic therapies may aim to address more than one mechanism involved in the control of airway mucus secretion and clearance.

DNase, gelsolin and acetylcysteine derivatives are all similar in action in that they degrade the three-dimensional network by mucolysis, or molecular weight disruption. This tends to preferentially affect the elasticity components of the network (as opposed to viscosity), which in model studies improve cough or airflow clearance more than clearance by ciliary action. Agents that affect ionic charge interactions and hydrogen bonds, on the other hand, are not true mucolytic agents because they alter the crosslink density without reducing polymer chain length, the result of which is common reduction in both elasticity and viscosity, and a preferential improvement in ciliary clearance according to model studies.

Ionic agents such as sodium chloride are believed to be mucoactive by shielding the fixed charges along the macromolecular core of the mucin polymer, making it less stiff and less extended and thus reducing the number of entanglement crosslinks with neighbouring macromolecules. Wills et al (J Clin Invest 1997; 99: 9-13) disclose that the degree of crosslink reduction is related to the ionic strength in the range of 0 to 500 mOsm NaCl. Nonionic agents such as sugar have also been suggested to improve mucus clearance by increasing osmolarity. Wills et al (J Clin Invest, supra) disclose that increasing the sputum osmolarity by addition of non-electrolytes such as glucose, mannitol and urea increases the ciliary transportability. PCT publication no. WO 95/22993 published on August 31, 1997 similarly discloses increasing mucociliary clearance by inhalation of a substance capable of altering the osmolarity of airway surface liquid, including sugar. On the other hand, PCT publication no. WO 95/28944 published on November 2, 1995 discloses that non-ionizable material such as glucose are not effective in improving sputum transportability.

WO 91/15216 discloses the use of polysulfated polysaccharides, including polysulfated dextran, for the treatment of human leucocyte elastase-medicated diseases.

Dextran is a bacterial byproduct; the dextran macromolecule consists of end-to-end linked glucan groups. Its primary clinical uses are as a plasma volume expander and as an antithrombotic agent which has antiaggregation effects. Dextran has also been shown to exhibit antiadhesive properties in airway epithelial cells, which may make it of value as an antimicrobial agent in preventing the Pseudomonas infection in CF patients (U.S. Patent No. 5,514,665 issued May 7, 1996 to Speert et al; Barghouthi et al Am J Respir Crit Care Med 1996; 154: 1788-1793).

It has now been found that dextran decreases mucus viscoelasticity and increases mucociliary clearability. The present invention relates to this unexpected finding that dextran and other polysaccharides may be used to improve mucus clearance.

## SUMMARY OF THE INVENTION

In one aspect, this invention relates to the use of a non-sulfated polysaccharide in the manufacture of a medicament to improve mucus clearance.

In another aspect, this invention relates to the use of a non-sulfated polysaccharide in the manufacture of a medicament to treat lung disease associated with impaired mucus clearance.

In one aspect, this invention finds application in a method of improving mucus clearability in a patient having cystic fibrosis comprising administering to the respiratory tract of said patient in need of such treatment an effective amount of dextran.

Preferably, the molecular weight of polysaccharide administered will be less than about 500,000, and more preferably less than about 250,000, most preferably less than about 2000.

## BRIEF DESCRIPTION OF THE DRAWINGS

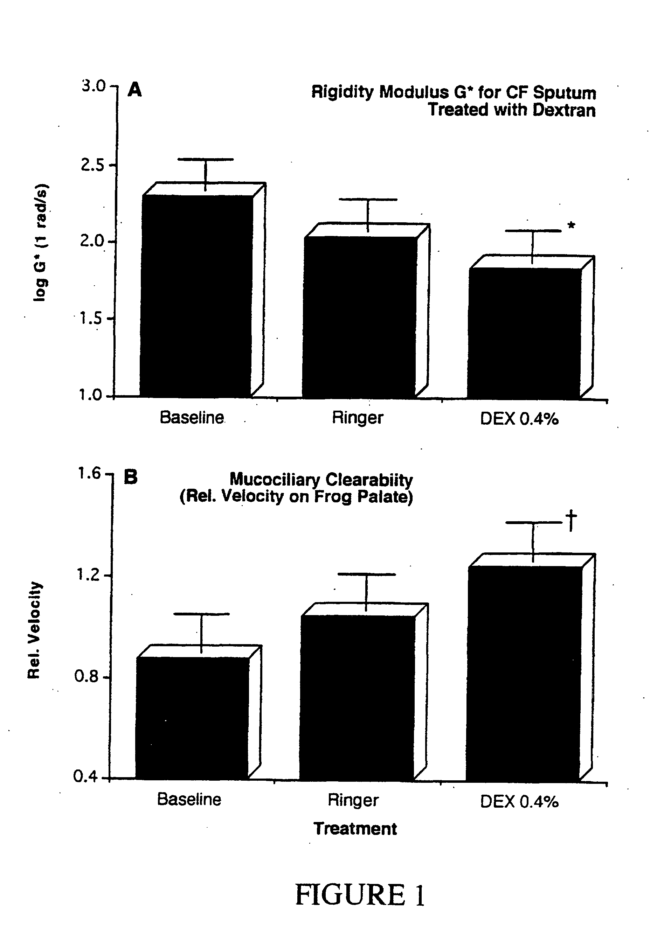

Figure 1A is a graph showing the effect of dextran treatment on viscoelasticity of sputum samples from CF patients. Figure 1B is a graph showing the effect of dextran treatment on mucociliary clearability of sputum samples from CF patients. Sputum samples were obtained from 7 CF patients not on rhDNase therapy. Aliquots of sputum were incubated at 37° for 30 minutes with either Ringer (10% added volume) or USHERDEX 4 (DEX) to achieve a final concentration of 0.4%. Rigidity modulus (viscoelasticity) was determined by magnetic rheometry, and mucociliary clearability was determined by observing the rate of sputum transport on excised frog palate. (* significantly different from Baseline; ÷ significantly different from Ringer.)

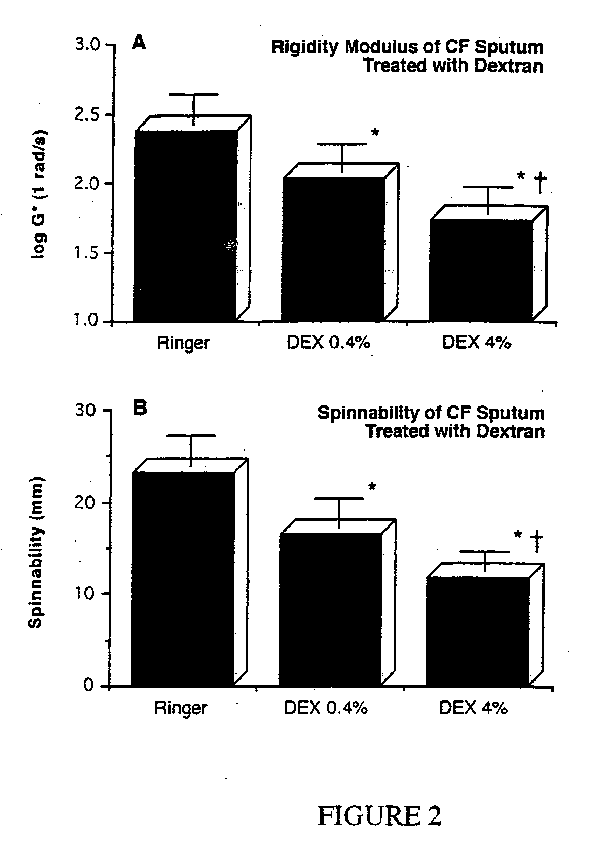

Figure 2A is a graph showing the effect of .4% and 4% dextran treatment on viscoelasticity of sputum samples from CF patients. Figure 2B is a graph showing the effect of .4% and 4% dextran treatment on spinnability of sputum samples from CF patients. Sputum samples were obtained from 8 CF patients riot receiving rhDNase. Aliquots of sputum were incubated at 37° for 30 minutes with Ringer (10% added volume) or USHERDEX 4 (DEX) to achieve final concentrations of 0.4% (4mg/ml) or 4% (40 mg/ml). Rigidity modulus (viscoelasticity) was determined by magnetic rheometry, and spinnability was determined by filancemeter. (* significantly different from Ringer; ÷ significantly different from DEX 0.4%.)

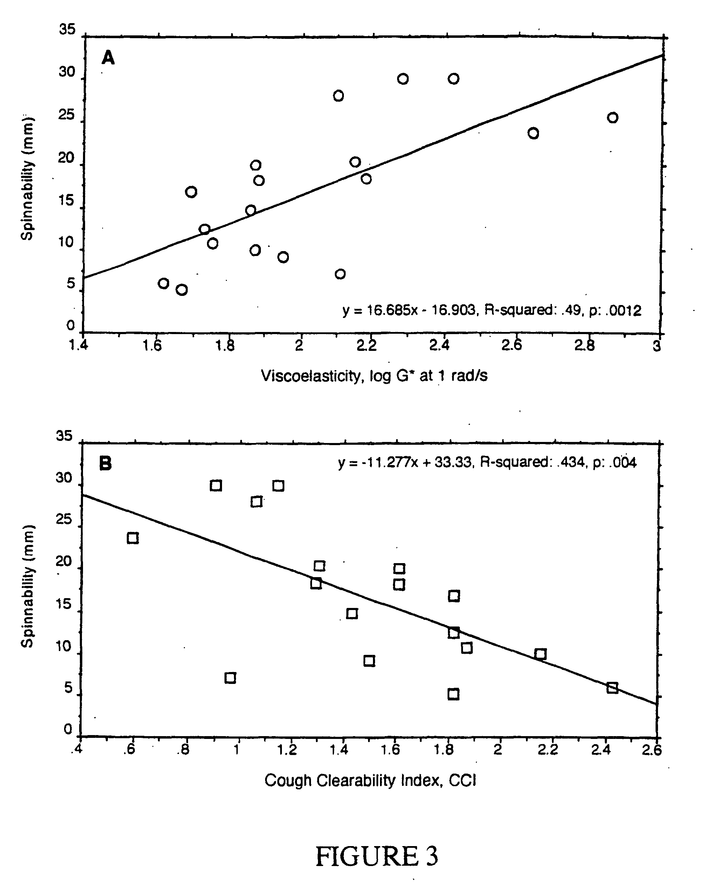

Figure 3A shows the correlation between spinnability and viscoelasticity. Figure 3B shows the correlation between spinnability and cough clearability. Sputum spinnability was determined by filancemeter, and rigidity modulus (viscoelasticity) was determined by magnetic rheometry, and cough clearability was predicted from viscoelastic data. A negative correlation between spinnability and MCI (mucociliary clearability index) was also obtained (p=.0138).

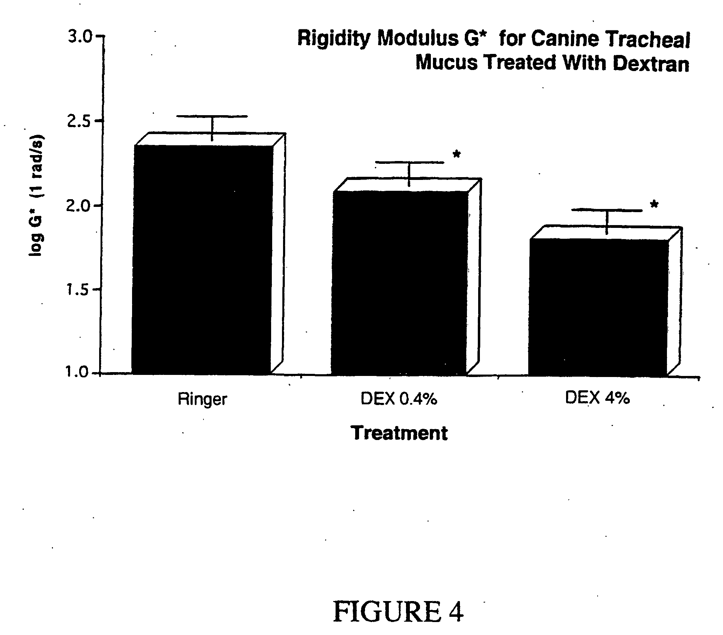

Figure 4 is a graph showing the effect of .4% and 4% dextran treatment on viscoelasticity of mucus obtained from healthy dogs. Tracheal mucus was obtained from 7 healthy, anaesthetized dogs from the endotracheal tube cuff. Aliquots of mucus were incubated at 37° for 30 minutes with Ringer (10% added volume) or USHERDEX 4 (DEX) to achieve final concentrations of 0.4% (4mg/ml) or 4% (40 mg/ml). Rigidity modulus (viscoelasticity) was determined by magnetic rheometry. (* significantly different from Ringer treatment.)

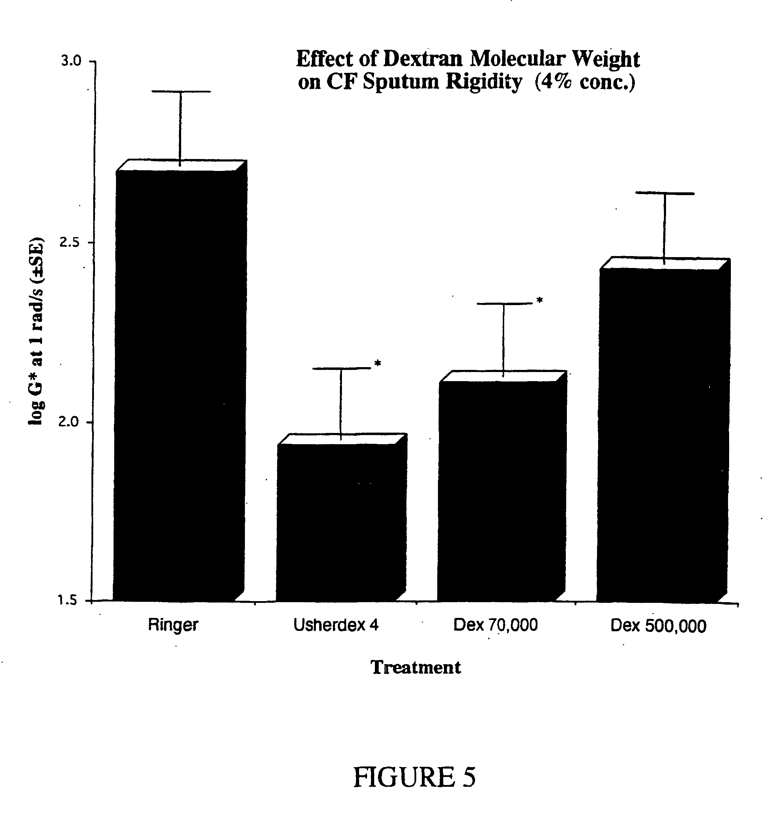

Figure 5 is a graph showing the effect of different molecular weights of dextran on viscoelasticity of sputum samples from CF patients. (* significantly different from Ringer treatment.)

DETAILED DESCRIPTION OF THE INVENTION

In the present invention, dextran has been shown to reduce viscoelasticity and increase mucociliary clearability of sputum obtained from CF patients *in vitro,* and the effects were dose dependent. Administration of 4% and . 4% dextran resulted in decreased spinnability and viscoelasticity, and the effects were greater at the higher dextran concentration. The predicted mucociliary clearability and cough clearability improved significantly at both concentrations and no significant dose dependency was observed with these derived parameters. The treatment of one CF patient with dextran resulted in decreased sputum viscosity and increased the predicted mucociliary clearability and cough clearability.

These effects of dextran are not specific to CF lung disease and dextran significantly reduced viscoelasticity of healthy dog mucus both *in vitro* and *in vivo.* The present invention is therefore not limited to improving mucus clearability in CF patients and may be used to treat other conditions associated with defect in airway mucus clearance including chronic bronchitis, bronchiectasis, bronchial asthma, mucus retention, mucus hypersecretion, and reduced mucociliary clearance.

The effects of dextran, a neutral polymer, are similar to those of sodium chloride treatment since both forms of treatment reduce sputum viscoelasticity *in vitro.* However, dextran of the present invention is believed to reduce viscoelasticity by competing for hydrogen bonding sites with other mucus glycoproteins resulting in the substitution, by dextran carbohydrate moieties, of oligosaccharide moieties linked to the high molecular weight mucin peptides that make up the mucus gel. For the lower molecular weight dextran (most preferably in the range of 2000 or less), these new hydrogen bonds are structurally and rheologically ineffective, thus reducing the overall crosslink density. This reduction in the crosslinking of the three dimensional mucus glycoprotein network is believed to improve mucus clearance by ciliary and cough mechanisms.

Dextran may be administered to the respiratory tract in the known manner, including by nebulizer via endotracheal tube, or topically, directly to the mucosa. Dextran may be administered in admixture with a pharmaceutically acceptable diluent or excipient. Acceptable diluents or excipients include sodium chloride or Ringer solution, L-arginine, or lysine and their salts. Preferably, dextran with a molecular weight range of about 360 to 4000 will be administered. Dextran at a final concentration of 4mg/ml and 40 mg/ml in the respiratory secretion may achieve the desired therapeutic effect. The concentration of 4mg/ml dextran in the respiratory secretion is achievable with aerosol delivery of 80 mg/ml dextran solution, based on model calculations. Dextran may be administered two to three times daily and the frequency will depend upon the individual patient's requirement. Other suitable dosage, frequency and modes of administration will be apparent to one skilled in the art.

The mechanism for the improvement in viscoelasticity with dextran administration is believed to be due to the substitution of dextran moieties in hydrogen bonding sites otherwise occupied by oligosaccharide moieties linked to neighbouring high molecular weight peptides. The original intermolecular mucin-mucin bonds contribute to the three-dimensional structure that makes up the mucus gel, while the new mucin-dextran bonds form ineffective crosslinks because of the relatively small length of the dextran polymer. Other polysaccharides with a similar number of hydrogen bonding sites to dextran of the present invention or containing sugar moieties that stereochemically complement the oligosaccharide moieties native to the respiratory tract mucins, including oligomers of galactose and fucose and the amino sugars glucosamine and galactosamine are expected to compete for hydrogen bonding sites in the mucus gel, by forming complementary interaction with the oligosaccharide side chains of mucin macromolecules and thereby reduce the overall crosslink density of the mucus gel. These polysaccharides, at a suitable dosage may be administered in the known manner to patients suffering from conditions associated with defect in the airway mucus clearance, including CF, chronic bronchitis, bronchiectasis and bronchial asthma.

The present invention will be further apparent from the following experiments. Moreover, one skilled in the art can readily appreciate that various modifications can be made to the described embodiments without departing from the scope and spirit of the invention. Such modifications are also intended to be within the scope of the invention.

**EXAMPLE 1**

Materials and Methods:

Subjects - Sputum samples were obtained by voluntary expectoration from 15 adolescent and young adult patients with CF. The patients were all infected with Pseudomonas aeruginosa. None of the patients was under current treatment with rhDNase. Approval to collect and use sputum for this in vitro analysis was obtained from the University of Calgary Research Ethics Board.

Dog mucus was obtained from healthy, anaesthetized dogs from the endotracheal tube cuff. Dextran (USHERDEX 4[*]) was provided by Polydex Pharmaceuticals Ltd. (Toronto, ON, Canada). USHERDEX 4 has been analyzed

[*] Trade-mark

by HPLC and contains dextran oligomers from two units (dimer) to 19 units (19-mer). USHERDEX 4 dextran has a molecular weight of range of about 360 to about 4000.

Study Design - Protocol A (CF sputum - 0.4% dextran):

Seven samples of sputum (0.40 to 0.60 g) were treated as follows:

i) Baseline aliquot (no in vitro treatment),
ii) Negative control aliquot, adding 10% vol. Ringer solution and incubating for 30 min. at 37° C.
iii) Application of 10% vol. Ringer's containing 40 mg/mL USHERDEX 4 to give a final concentration of 0.4% (4 mg/mL) in the sputum; the sample was incubated for 30 min. at 37° C.

Protocol B (CF sputum dose-response):

Eight samples of sputum (0.40 to 0.60 g) were treated as follows:

i) Negative control aliquot, adding 10% vol. Ringer solution and incubating for 30 min. at 37° C.
ii) Application of Ringer containing 40 mg/mL USHERDEX 4 to give a final concentration of 0.4% (4 mg/mL) in the sputum.
iii) Application of Ringer containing 400 mg/mL USHERDEX 4 to give a final concentration of 4% (40 mg/mL) in the sputum.

Protocol C (canine tracheal mucus dose-response):

Seven samples of canine tracheal mucus (ca. 0.1 g) were treated as follows:

i) Negative control aliquot, adding 10% vol. Ringer, incubated 30 min. at 37°C.
ii) Application of Ringer containing 40 mg/mL USHERDEX 4 to give a final concentration of 0.4% (4 mg/mL) in the sputum, incubated 30 min. at 37° C.
iii) Application of Ringer containing 400 mg/mL USHERDEX 4 to give a final concentration of 4% (40 mg/mL) in the sputum, incubated 30 min. at 37° C.

Frog palate mucociliary transportability (FMT): This was determined from CF sputum movement on the ciliated, mucus-depleted frog palate, relative to native frog control (rel.vel., FMT, expressed as a fraction). The movement of a 2-5 μL aliquot of CF sputum was timed; 5 measurements of sputum transport rate were taken to minimize measurement variability. FMT was determined only for the samples in protocol A.

Rheological Measurements on CF Sputum: In this in vitro study, two techniques were used to measure the rheological properties of sputum: spinnability by filancemeter and viscoelasticity by magnetic rheometry.

Spinnability is the thread forming ability of mucus under the influence of low amplitude elastic deformation. The spinnability of CF sputum samples was measured using a Filancemeter (SEFAM, Nancy, France), in which a 20 to 30 μL mucus sample is stretched at a distraction velocity of 10 mm/s. An electric signal conducted through the mucus sample is interrupted at the point where the mucus thread is broken. The length of this thread is known as the mucus spinnability (measured in mm). Spinnability was only determined for sputum samples in protocol B.

Viscoelasticity and Clearance Indices: The magnetic microrheometer technique was used to measure the viscosity and elasticity of the sputum samples. A 100 μm steel ball was positioned in a 5-10 μL sample of sputum, and the motion of this sphere under the influence of an electromagnet was used to determine the rheological properties of the sputum. The image of the steel ball was projected via a microscope onto a pair of photocells, whose output was amplified and transmitted to an oscilloscope. By plotting the displacement of the ball against the magnetic driving force, the viscoelastic properties of the mucus were ascertained.

The parameters of mucus viscoelasticity determined were the rigidity index or mechanical impedance, i.e. $G^*$, reported here on a log scale, expressing the vector sum of "viscosity + elasticity". Two derivative parameters - mucociliary clearability index (MCI) and cough clearability index (CCI) - were computed from in vitro relationships. These two indices predict mucus clearability by ciliary and cough mechanisms, respectively, based on the measured rheological properties and observations of clearance from model studies. The respective formulas are as follows:

$$MCI = 1.62 - (0.22 \times \log G^* 1) - (0.77 \times \tan \delta 1)$$

$$CCI = 3.44 - (1.07 \times \log G^* \ 100) + (0.89 \times \tan \delta \ 100)$$

Statistical Analysis - Data from each protocol are presented as mean ± standard deviation (SD) of the mean. To analyse the significance of changes in spinnability, log G* at 1 rad/s, MCI, and CCI after administration of Ringer control, 0.4% dextran and 4% dextran, the sputum from each patient served as its own control. Equality of means was tested by analysis of variance (ANOVA), post hoc analysis of changes from baseline was determined by the two-tailed, paired t-test and regression. The paired t-test was also used to determine the differences between spinnability and viscoelasticity after different treatments. Regression was used to determine the correlation between spinnability and viscoelasticity. The StatView statistical package (Abacus Concepts, Palo Alto, CA) was used to carry out these analyses.

Results:

In protocol A, after administration of 0.4% USHERDEX 4, frog palate mucociliary clearability increased significantly (p=.046) compared to Ringer control (Figure 1B). There was a modest dilution effect (reduction in mucus rigidity) associated with the Ringer treatment, and a further reduction due to the Dextran (significant w.r.t. no treatment, p=.004) (Figure 1A). Cough clearability (CCI, predicted from rheology) also increased significantly (p=.019). Mucociliary clearability on frog palate increased more than that predicted from rheology, this extra clearability could be an indication of a surface or antiadhesive effect.

After the initial tests suggested that USHERDEX 4 might have direct, potentially beneficial effects on CF sputum, we conducted further experiments to investigate the dose-dependency of the effects (protocol B) and whether noninfected mucus was also influenced by USHERDEX 4 (protocol C).

In protocol B, compared to saline control, CF sputum spinnability decreased 34.2% by administration of 0.4% dextran (p=.0121) and 59.8% by administration of 4% dextran (p=.0016) (Figure 2B). Furthermore, the reduction in spinnability after 4% dextran was greater than that seen after 0.4% (p=.0046). At the same time, mucus viscoelasticity (log G*1) was reduced by a factor of 2.43 (0.385 log units) by 0.4% dextran (p=.0404) and by a factor of 4.57 (0.660 log units) by 4% dextran (p=.0069) (Figure 2A). The additional reduction in mucus viscoelasticity observed at the higher dextran concentration was also significant (p=.0193). Mucociliary clearability (MCI) and cough clearability (CCI) (both predicted from rheology) improved significantly in both treatment groups (MCI: p=.0252, p=.003; CCI: p=.0227, p=.0423), although no significant dose dependency was seen with these derived parameters. There was a positive relationship between spinnability and viscoelasticity (Figure 3A), as well as negative correlations between spinnability and predicted mucociliary clearance and cough clearance (Figure 3B).

In protocol C, the viscoelasticity of healthy dog mucus was decreased significantly by treatment with 0.4% dextran ($\partial$ log G* = 0.269, p=.0048) and with 4% dextran ($\partial$ log G* = 0.547, p=.0016) compared to saline control (Figure 4). Mucociliary clearability (predicted from rheology) only increased significantly for 4% dextran (p=.0108). Cough clearability (predicted from rheology) increased significantly in both treatment groups (p=.0385; p=.0459). The additional reduction in log G* between 0.4% and 4% dextran treatments did not achieve statistical significance (p=.09).

Overall, whether for CF sputum or healthy dog mucus, USHERDEX 4 treatment significantly reduced viscoelasticity and increased predicted mucociliary and cough clearability. This effect of dextran was dose dependent, being significantly greater for 4% dextran treatment than for 0.4%. There was no great correlation between frog palate mucus transportability and mucus viscoelasticity, but there was a significant correlation between spinnability and mucus viscoelasticity (p=.0012), spinnability and mucociliary clearability (p=.0138), as well as cough clearability (p=.004) (both predicted from rheology) (Figure 3).

**EXAMPLE 2**

• The Effect of Dextran Molecular Weight on CF Sputum Rigidity:

Method:

Sputum samples were obtained from 8 CF patients not receiving rhDNase (Pulmozyme®)-Aliquots of sputum were incubated at 37° for 30 minutes with Ringer diluent (10% added volume) or with three dextrans of different molecular weights, i.e. USHERDEX 4 (average molecular weight of 1800), Dextran 70,000 and Dextran 500,000, to achieve a final concentration of 4% (40 mg/ml). The rigidity modulus (viscoelasticity) was determined by magnetic rheometry.

Results:

The highest molecular weight dextran had no significant effect on mucus viscoelasticity, while the effect of USHERDEX 4 is comparable to that previously shown in Example 1. The intermediate fraction had an intermediate effect (Figure 5).

**EXAMPLE 3**

• Effects of Dextran on Dog Mucus

Method:

Healthy mongrel dogs were anaesthetized with pentobarbital and intubated. After a 30 min. Ringer aerosol, tracheal mucociliary velocity (TMV by charcoal particle transport) was measured under bronchoscopic control, and mucus for viscoelasticity analysis (magnetic rheometer) was collected by the endotracheal tube method. Then low m. w. dextran (Polydex Pharma., lot #2764, molecular weight range of about 360 to about 4000) in Ringer vehicle was aerosolized to the trachea, followed by the same procedures. We performed 8 expts. in 8 dogs, involving dextran aerosol 30 min administration; all dogs received aerosols of 20 mg/ml, 65 mg/ml, 200 mg/ml dextran.

• Results:

Compared with Ringer control, TMV increased to 145% of control (p=.0417) at 65 mg/ml dextran. There was only a modest increase in TMV at 20 mg/ml and a decrease in TMV with 200 mg/ml dextran. Mucus viscoelasticity significantly decreased to 35% of control (0.452 log units, p=.0426) at 65 mg/ml. These *in vivo* data support *in vitro* findings, that low molecular weight dextran deceases mucus viscoelasticity and increases mucociliary clearance.

**EXAMPLE 4**

• Interaction of Dextran with L-Arginine on Tracheal Mucus Secretion and Clearance in Dogs

Method:

Arginine/arg. HCl was prepared as described by Solomons (Pediatrics 1971;47:384), making a stock solution of 290 mOsm, pH 7.4. Two dilutions were prepared, 97 and 29 mOsm, the balance being Ringers. The solutions were delivered by Pari jet nebulizer to 8 healthy mongrel dogs anaesthetized with pentobarbital and intubated. After 30 min. Ringer aerosol, tracheal mucus velocity (TMV by charcoal particle transport) and potential difference (PD by agar bridge) were measured under bronchoscopic control, and mucus for viscoelasticity analysis (magnetic rheometer) was collected on the endotracheal tube. On one occasion, increasing arginine conc. were aerosolized hourly, following Ringer control. On a second occasion, the same arginine solutions were used, but with 65 mg/ml low m.w. dextran (Polydex, lot #2764) added.

Results:

Buffered L-arginine has been described as a mucolytic treatment (Solomons, Pediatrics 1971; 47:384), although its effectiveness remains uncertain. However, arginine is a precursor of nitric oxide, a critical mediator in many cellular processes including mucus secretion, and there is evidence that arginine and the related amino acid lysine can stimulate transepithelial water flux via apical chloride channels, thereby increasing airway fluid hydration. Indeed, when lysine is added to the classical mucolytic N-acetylcysteine, the mucokinetic effect is enhanced, at least in part, through stimulation of epithelial Cl transfer (Tomkiewicz, Pulm Pharm 1995;8:259). In view of these considerations, we decided to study the combination of arginine and dextran to see if the mucokinetic effect of the dextran could be enhanced by coadminstration of arginine, or indeed if arginine itself had significant mucolytic activity. Compared with Ringer, the lumen-negative PD decreased progressively with increasing arginine, with or without added dextran (from - 14.3 ± 1.4 mV to -20.1 ± 3.6 mV), TMV increased on avg., but nonsignificantly (11.9 ± 2.2 to 14.7 ± 2.9 mm/min), while mucus rigidity (log G*) decreased for dextran treatment (2.09 ±.13 to 1.79 ±.07) but not for arginine. Our results do not support the use of arginine as a mucolytic agent by itself, nor does addition of arginine appear to augment the mucolytic activity of dextran, at least in healthy animals. However, the decrease in PD is consistent with the stimulation of transepithelial ion flux, which suggests a potentially positive effect in terms of airway fluid hydration and arginine might have a greater effect in a model of chronic bronchitis or CF.

**EXAMPLE 5**

With the permission of the Health Protection Branch of the Ministry of Health and Welfare Canada, one patient with advanced CF lung disease was treated with dextran. The patient was already receiving aerosolized L-arginine and dextran was administered in L-arginine. The following formulation was used: 200 mg dextran (molecular weight range of about 360 to about 4000) per ml of L-arginine solution (50 g L-arginine HCl and 3 g arginine free base per litre water: Solomons, 1971). No adverse effect was observed with a test dose of 200 mg/ml for 30 seconds and a .5 g dose was administered over eight minutes by nebulizer via endotracheal tube. This initial treatment resulted in the production of copious liquid secretions and the treatment was repeated three times a day over the next two days. There was evidence of liquification of secretions with each treatment. The treatment was stopped because the patient suffered a sudden episode of pulmonary decompensation.

Sputum viscoelasticity was assessed on samples obtained before and after the treatment by magnetic rheometry. The results obtained are shown in the following Table 1.

Table 1

|   | Date | Time | Treatment | logG*1 | tand 1 | MCI | CCI | Comment |
|---|------|------|-----------|--------|--------|-----|-----|---------|
| 1 | 97.10.17 | 20:45 | pre-dextran | 1.775 | .505 | .845 | 1.490 | very purulent sample |
| 2 | 97.10.17 | 23.15 | post-dextran | 1.650 | .360 | .980 | 2.460 | |
| 3 | 97.10.20 | 16.10 | post-dextran | 2.520 | .290 | .890 | • | |

After the first treatment, elasticity deceased modestly, while viscosity decreased more substantially, and predicted mucociliary clearability and cough clearability both increased. Two days later, after the last treatment, viscosity decreased to a normal or lower than normal value while elasticity was higher and the predicted mucociliary clearability was higher than before treatment.

**EXAMPLE 6**

The effects on frog palate mucociliary clearance of two solutions, labeled A and B. A was a 4% (40 mg/mL) solution of dextran lot 1352 in amphibian Ringer's (R). B was a 4% solution of nominal 10,000* MW dextran. Leopard frogs (rana pipiens) were chilled and pithed; the palate was excised and viewed under a macroscope in a humidified chamber. To test for mucus clearability, 1.25 µL of solution was applied to the midline of the exposed palate, and measurements of marker particle clearance were made over the subsequent 10 minute period (mean of 7 observations). The order of application was alternated, ie. A, R, B or B, R, A, to account for time effects.

Three valid paired results were obtained from three freshly excised frog palates:

Velocity (mm/min):

| palate 1: A | 18.31 | | R | 12.72 | B | 15.60 |
|---|---|---|---|---|---|---|
| palate 2: B | 23.56 | | R | 22.84 | A | 29.07 |
| palate 6: A | 17.41 | | R | 14.17 | B | 14.99 |
| ratios: A/R: | 1.439 | 1.273 | 1.229 | | mean(3) = 1.314 | |
| B/R | 1.226 | 1.032 | 1.058 | | mean(3) = 1.105 | |
| A/B | 1.174 | 1.234 | 1.161 | | mean(3) = 1.190 | |

The conclusion: solution A (dextran lot 1352 stimulates mucociliary clearance by about 20% more than solution B. Our previous results indicated that the same concentrations of glucose dimer, trimer or tetramer were of intermediate effectiveness (9 to 19% stimulation over control).

**Claims**

**1.** Use of a non-sulfated polysaccharide in the manufacture of a medicament to improve mucus clearance.

**2.** The use as claimed in claim 1 wherein the non-sulfated polysaccharide is dextran.

* This lot in fact has an average molecular weight of 2200 as determined by HPLC. Lot 1352 has an average molecular weight of 1600 as determined by HPLC.

3. The use as claimed in claim 2, wherein the dextran is in admixture with a pharmaceutically acceptable diluent or carrier.

4. The use as claimed in claim 3, wherein the diluent is sodium chloride or Ringer solution.

5. The use as claimed in claim 2, wherein the dextran is to be administered topically or by aerosol.

6. The use as claimed in any one of claims 2 to 5, wherein the dextran has an average molecular weight of less than 500,000.

7. The use as claimed in any one of claims 2 to 5, wherein the dextran has a molecular weight range of about 360 to about 4000.

8. The use as claimed in any one of claims 2 to 5, wherein the dextran has a molecular weight range of less than about 2000.

9. The use as claimed in any one of claims 2 to 8, wherein the dextran is present in the respiratory secretion at a concentration of about 4 mg/ml to about 40 mg/ml.

10. The use as claimed in claim 1 wherein the non-sulfated polysaccharide is a polysaccharide with a similar number of hydrogen bonding sites to dextran or containing sugar moieties that stereochemically complement the oligosaccharide moieties native to the respiratory tract mucins, including oligomers of galactose and fucose and the amino sugars glucosamine and galactosamine, in the manufacture of a medicament to improve mucus clearance.

11. Use of a non-sulfated polysaccharide in the manufacture of a medicament to treat lung disease associated with impaired mucus clearance.

12. The use as claimed in claim 11 wherein the non-sulfated polysaccharide is dextran.

13. The use as claimed in claim 11 or 12, wherein the lung disease is cystic fibrosis, chronic bronchitis, bronchiectasis or bronchial asthma.

14. The use as claimed in claim 11 or 12, wherein the disease is cystic fibrosis.

**Patentansprüche**

1. Verwendung eines nicht-sulfatierten Polysaccharids zur Herstellung eines Medikaments zur Förderung der Mucus-Clearance.

2. Verwendung nach Anspruch 1, wobei das nicht-sulfatierte Polysaccharid Dextran ist.

3. Verwendung nach Anspruch 2, wobei das Dextran in einer Mischung mit einem pharmazeutisch verträglichen Verdünnungsmittel oder Träger vorliegt.

4. Verwendung nach Anspruch 3, wobei das Verdünnungsmittel Natriumchlorid oder Ringer-Lösung ist.

5. Verwendung nach Anspruch 2, wobei das Dextran topisch oder durch Aerosol zu verabreichen ist.

6. Verwendung nach einem der Ansprüche 2 bis 5 , wobei das Dextran ein durchschnittliches Molekulargewicht von weniger als 500.000 besitzt.

7. Verwendung nach einem der Ansprüche 2 bis 5, wobei das Dextran einen Molekulargewichtsbereich von etwa 360 bis etwa 4.000 besitzt.

8. Verwendung nach einem der Ansprüche 2 bis 5, wobei das Dextran einen Molekulargewichtsbereich von weniger als etwa 2.000 besitzt.

**9.** Verwendung nach einem der Ansprüche 2 bis 8, wobei das Dextran in dem Atemwegssekret in einer Konzentration von etwa 4 mg/ml bis etwa 40 mg/ml vorhanden ist.

**10.** Verwendung nach Anspruch 1, wobei das nicht-sulfatierte Polysaccharid ein Polysaccharid mit einer ähnlichen Anzahl von Wasserstoffbindungsstellen wie Dextran ist oder Zuckerbausteine enthält, welche die Oligosaccharidbausteine stereochemisch ergänzen, die natürlich in den Mucinen der Atemwege vorkommen, einschließlich der Oligomere von Galactose und Fucose und der Aminozucker Glucosamin und Galactosamin, zur Herstellung eines Medikaments zur Verbesserung der Mucus-Clearance.

**11.** Verwendung eines nicht-sulfatierten Polysaccharids zur Herstellung eines Medikaments zur Behandlung einer Lungenkrankheit, die mit einer verschlechterten Mucus-Clearance verbunden ist.

**12.** Verwendung nach Anspruch 11, wobei das nicht-sulfatierte Polysaccharid Dextran ist.

**13.** Verwendung nach Anspruch 11 oder 12, wobei die Lungenkrankheit cystische Fibrose, chronische Bronchitis, Bronchiektasie oder bronchiales Asthma ist.

**14.** Verwendung nach Anspruch 11 oder 12, wobei die Krankheit cystische Fibrose ist.


**Revendications**

**1.** Utilisation d'un polysaccharide non sulfaté dans la fabrication d'un médicament pour améliorer la clairance du mucus.

**2.** Utilisation selon la revendication 1, dans laquelle le polysaccharide non sulfaté est le dextrane.

**3.** Utilisation selon la revendication 2 , dans laquelle le dextrane est en mélange avec un diluant ou un support pharmaceutiquement acceptable.

**4.** Utilisation selon la revendication 3, dans laquelle le diluant est une solution de chlorure de sodium ou la solution de Ringer.

**5.** Utilisation selon la revendication 2, dans laquelle le dextrane doit être administré localement ou par aérosol.

**6.** Utilisation selon l'une quelconque des revendications 2 à 5, dans laquelle le dextrane a un poids moléculaire moyen inférieur à 500 000.

**7.** Utilisation selon l'une quelconque des revendications 2 à 5, dans laquelle le dextrane présente une plage de poids moléculaire d'environ 360 à environ 4000.

**8.** Utilisation selon l'une quelconque des revendications 2 à 5, dans laquelle le dextrane a un poids moléculaire inférieur à environ 2000.

**9.** Utilisation selon l'une quelconque des revendications 2 à 8, dans laquelle le dextrane est présent dans la sécrétion respiratoire à une concentration d'environ 4 mg/ml à environ 40 mg/ml.

**10.** Utilisation selon la revendication 1, dans laquelle le polysaccharide non sulfaté est un polysaccharide comportant un nombre de sites de fixation d'hydrogène semblable au dextrane ou contenant des restes sucres qui complètent stéréochimiquement les restes oligosaccharides inhérents aux mucines du tractus respiratoire, y compris les oligomères de galactose et de fucose et les aminosucres glucosamine et galactosamine, dans la fabrication d'un médicament pour améliorer la clairance du mucus.

**11.** Utilisation d'un polysaccharide non sulfaté dans la fabrication d'un médicament pour traiter une maladie pulmonaire liée à une anomalie de la clairance du mucus.

**12.** Utilisation selon la revendication 11, dans laquelle le polysaccharide non sulfaté est le dextrane.

13. Utilisation selon la revendication 11 ou 12, dans laquelle la maladie pulmonaire est la mucoviscidose, la bronchite chronique, la bronchectasie ou l'asthme bronchique.

14. Utilisation selon la revendication 11 ou 12, dans laquelle la maladie est la mucoviscidose.

FIGURE 1

FIGURE 2

FIGURE 3

14

Rigidity Modulus G* for Canine Tracheal
Mucus Treated With Dextran

FIGURE 4

FIGURE 5